# EUROPEAN PATENT APPLICATION

(11) **EP 0 745 597 A2**
(43) Date of publication of application: **04.12.1996**
(21) Application number: 96401088.8
(22) Date of filing: 17.05.1996
(51) Int. Cl.: C07D 295/18, C07D 333/38, C07D 307/68, C07D 295/20, C07D 207/40, C07D 209/42, C07D 285/06, A61K 31/495

(54) **Novel melatonergic indanyl piperazines or homopiperazines**

(30) Priority: 02.06.1995 US 458924; 02.06.1995 US 458925
(71) Applicant: BRISTOL-MYERS SQUIBB COMPANY, Princeton, NJ 08543-4000 (US)
(72) Inventor: Mattson, Ronald J., Meriden, CT 06450 (US); Sloan, Charles P., Wallingford, CT 06492 (US); Catt, John D., Southington, CT 06489 (US)
(74) Representative: Beauchamps, Georges

(57) **Abstract**

Certain indanyl piperazines are useful as melatonergic agents and can treat various CNS disorders. They are use useful in treating sleep disorders and other conditions related to circadian rhythms.

## Description

The invention deals with indanyl-substituted piperazines, also known in the literature as 1-(2,3-dihydro-1H-inden-1-yl)piperazines, which have bioaffecting properties and to their preparation, formulation and use. Specifically, the invention is concerned with highly water soluble piperazines having substituted indanyl moieties attached to one nitrogen of the piperazine ring. These compounds are useful melatonergic agents because they are ML₁ receptor agonists and partial agonists. They have potential activity as sedatives, for treating sleep-related disorders and for treating anxiety, depression and various CNS disorders related to circadian rhythms.

Melatonin, i.e., N-acetyl-5-methoxytryptamine, is a hormone which is synthesized and secreted primarily by the pineal gland. In mammals, melatonin levels show a cyclical, circadian pattern, with with highest levels occuring during the dark period of a circadian light-cark cycle. Melatonin appears to modulate a variety of neural and endocrine functions in vertebrates, including the regulation of reproduction, the control of circadian rhythms and the modulation of retinal physiology.

Melatonin binding sites have been found in several diverse tissues of the body-i.e., in the retina, superchiasmatic nucleus, spleen, etc. Thus, melatonin exerts multiple physiological effects, is not highly selective, and has a potential for producing unwanted side effects. Melatonin agonists should be more selective than melatonin and give fewer side effects, resulting in products having more predictable activity.

The melatonin antagonist, luzindole, exhibits antidepressant-like effects. See Dubocovich et al, European Journal of Pharmacology, 182,(1990), pages 313-325. Luzindole binds to human melatonin receptors. Like luzindole, compounds of this invention bind to these receptors and, therefore, are believed to have antidepressant character.

Certain compounds of the invention are structurally related to compounds disclosed as intermediates in Manoury, et al., U.S. patent 4,963,680. See Examples 2 through 5 of the patent. However, the compounds of the '680 patent are not taught as having therapeutic activity of their own. They are only discussed as chemical intermediates in processes for making chemically distinct compounds which are therapeutic agents.

90-242933/32,EISA 22.12.88, JO 2169-569-A shows cydic amine derivatives for the treatment or prophylaxis of senile dementia, cerebral apoplexy, cerebral atherosclerosis, traumatic cerebral damage, post cerebral edema, or cerebral palsy. The compounds can be piperidines or piperazines linked to carboxamide groups and to heterocyclic rings. 89-001045/01, EISA 22.06.87, EP 296-560-A shows similar compounds having selective antiacetylcholinesterace activity.

89-074668/10, TAIY-24.07.87, JO 1029-310-A shows asthma medicaments of formula i: 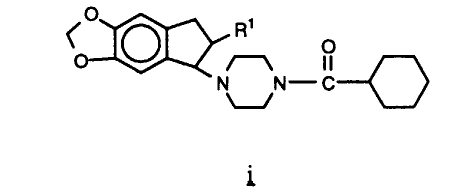 wherein R₁ is lower alkyl.

88-272140/39, TAIY-27.03.83, EP-283-551-A deals with drugs for amelioration of cerebral circulation and metabolism which are indene derivatives of formula ii: 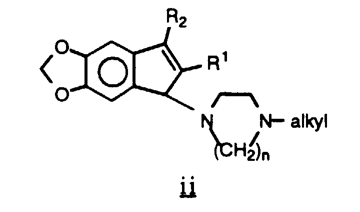 wherein R₁ is lower alkyl, R₂ is H, aryl, or lower alkyl and n is 2 or 3.

88-046878/07, TAIY-26.06.86, J6 3005-063-A refers to indanes used to treat bronchial asthma. The compounds are of formula iii:

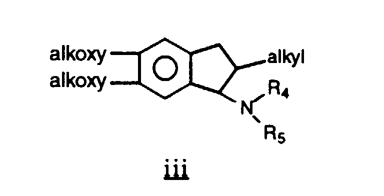

In formula iii, R₄ and R₅ may form, with the N atom, or piperidinyl, piperazinyl, or homopiperazinyl group.

U.S. 4,983,607 to Manoury, et al., discusses quinolinone derivatives which possess high affinity for "5-HT_{1A} type serotoninergic receptors".

The structurally related compound 1-(2,3-dihydro-6-methoxy-1H-inden-1-yl)-1-homopiperazine is disclosed in Example 2 of U.S. patent 4,963,680.

WO 92/10192 of Bogeso, et al., shows compounds of formula iv: 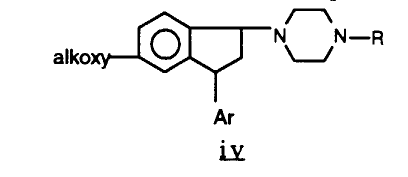 wherein Ar is an optionally substituted phenyl, thiophene or furan ring; and R can be hydrogen, alkyl, alkenyl, cycloalkyl, or cycloalkyl- substituted alkyl.

In addition, indanyl and tetralinyl-piperazines have been disclosed as useful therapeutic agents in a variety of patents, such as: US 5,010,079; WO 9322293-A1; EP 49772-A1; WO 9316057; EP 35363; EP 354093A; EP354094A; and WO 9210292. For example, compounds of general formula vi are claimed in EP 183,349A1, as potent 5-HT₂ antagonists useful in treatment of cardiovascular diseases including hypertension and anxiety.

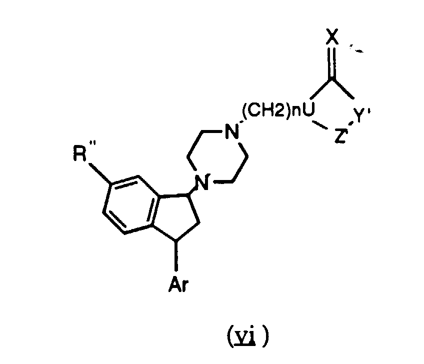

Also disclosed therein is the preparation of these compounds via Method a (below) by alkylation of a substituted or unsubstituted piperazine with a halo- or alkanesulfonyl-indane (III) to give the key intermediate, IV. The intermediate, III, is prepared from the corresponding indanones, II, by reduction of the ketone and subsequent conversion of the resulting alcohol to a halo- or alkanesulfonyl leaving group. One problem with this multi-step method is that conditions must be rigorously controlled to prevent the elimination of HX from the halo- or alkanesulfonyl-indane (III) to give the undesired indene byproduct, V, during both steps of this procedure.

Method a is:

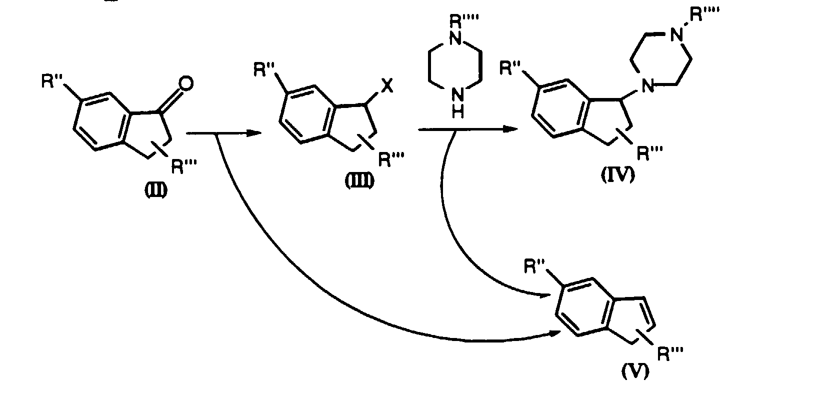

All of the publications cited in the last four paragraphs use synthetic strategies similiar to Method a.

In its broadest aspect, the invention is concerned with indanyl piperazines having useful melatonergic properties, their preparation, and methods and compositions which employ them.

The compounds of the invention are those of formula I and pharmaceutically acceptable salts thereof. Formula I is: 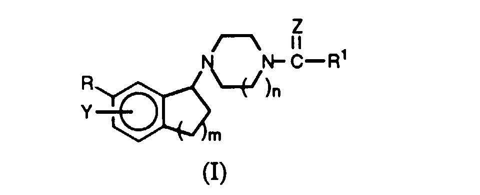 wherein:
R is H, C₁₋₄ alkyl, or C₁₋₉ alkoxy;
Y is hydrogen, C₁₋₄ alkoxy, or halogen;
Z is O or S;
m is 1 or 2;
n is 1 or 2; and
R¹ is C₁₋₆ alkyl (straight or branched), C₁₋₆ haloalkyl, C₁₋₄ alkoxy, C₁₋₄ thioalkoxy, C₁₋₄ thioalkoxy substituted C₁₋₄ alkyl, C₁₋₆ alkoxy substituted C₁₋₄ alkyl, C₂₋₂₀ alkenyl (straight or branched), C₃₋₆ cycloalkyl, phenyl, thienyl, pyrrolyl, furanyl, thiadiazolyl, indolyl, or NR²R³ wherein R² and R³ are independently hydrogen, C₁₋₃ alkyl or C₃₋₆ cycloalkyl.

Pharmaceutically acceptable salts, solvates or mixtures of these compounds can be used.

The compounds of the invention are advantageous in several ways. They have melatonergic and other CNS properties and are believed useful as agents for the treatment of stress, sleep disorders, seasonal depression, appetite regulation, shifts in circadian cycles (e.g. jet lag), melancholia and the like.

In addition, they are highly water soluble, penetrate the blood-brain barrier well and have long biological half-lives. Their physical and pharmacological properties make them excellent candidates for delivery via oral dosage forms.

In Formula I, R is H, C₁₋₄ alkyl or C₁₋₉ alkyloxy.

Y may be hydrogen, C₁₋₄ alkyloxy or halogen.

Z is O or S.

m is 1 or 2.

n is 1 or 2, with compounds in which n is 1 being preferred.

R¹ is C₁₋₆ alkyl (straight or branched), C₁₋₆ haloalkyl, C₁₋₄ alkoxy, C₁₋₄ thioalkoxy, (C₁₋₄ thioalkoxy) C₁₋₄ alkyl, (C₁₋₆ alkoxy) C₁₋₄ alkyl, C₂₋₂₀ alkenyl (straight or branched), C₃₋₆ cycloalkyl, phenyl, thienyl, pyrrolyl, furanyl, thiadiazolyl, indolyl, or NR²R³ wherein R² and R³ are independently hydrogen, C₁₋₃ alkyl or C₃₋₆ cycloalkyl.

By "alkyl" applicants mean straight or branched saturated acyclic moieties containing the indicated number of carbon atoms. In this disdosure, "Me", "Et", "n-Pr", "i-Pr", and "c-Pr" refer to CH3-, CH₃CH₂-, CH₃CH₂CH₂-, (CH₃)₂CH-, and 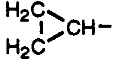 groups, respectively.

When using "haloalkyl", applicants mean alkyl groups bearing from one to three substituents selected from Br, Cl, F or I.

The term "alkoxy" refers to alkyloxy or -O-alkyl moieties.

By "alkenyl" is meant straight or branched moieties having from 2 to 4 carbon atoms and containing one site of ethylenic unsaturation.

The term "cycloalkyl" refers to saturated cyclic groups conforming to the formula CₓH(₂ₓ₋₁) and containing from 3 to 5 carbon atoms.

"NR²R³" refers to monoalkyl- and dialkyl-amino groups wherein R² and R³ are independently hydrogen, or noncyclic, straight, or branched C₁₋₄ alkyl or C₃₋₅ cycloalkyl moieties. Groups wherein R² is straight or branched C₁₋₄ alkyl and R³ is hydrogen are preferred.

By "halogen" is meant bromine, chlorine, fluorine or iodine substituents.

When R¹ is a group containing a cycloalkyl, phenyl, furanyl, thienyl, pyrrolyl or indolyl ring, one or more ring substituent(s) can be present. Suitable ring substituents are: alkyl (preferably methyl), hydroxyl, or halogen (preferably one or two bromine or chlorine atoms). Thus, R¹ may be chlorophenyl, hydroxyphenyl, bromofuranyl, methylfuranyl, methyl cyclohexyl, bromo- or chloro-thienyl, dibromothienyl and the like.

In chemical terms herein, "indane," "indanyl," "tetralin," and "tetralinyl" refer respectively to "2,3-dihydro-1H-indene," and "2,3-dihydro-1H-inden-1-yl," "1,2,3,4-tetrahydronaphthalene," and "1,2,3,4-tetrahydronaphthalen-1-yl."

Compounds of Formula I also encompass all pharmaceutically acceptable acid addition salts thereof. The pharmaceutically acceptable acid additions salts of the invention are those in which the counter-ion does not contribute significantly to the toxicity or pharmacological activity of the salt and, as such, they are the pharmacological equivalents of the bases of Formula I. They are generally preferred for medical usage. In some instances, they have physical properties which make them more desirable for pharmaceutical formulations. Such properties include solubility, lack of hygroscopicity, compressibility with respect to tablet formation and compatibility with other ingredients with which the substance may be used for pharmaceutical purposes. Salts of piperazines are highly water soluble.

The salts are routinely made by admixture of a Formula I base with the selected acids, preferably by contact in solution employing an excess of commonly used inert solvents, such as water, ether, benzene, methanol, ethanol, ethyl acetate, acetone, and acetonitrile. They may also be made by metathesis or treatment with an ion exchange resin under conditions in which the anion of one salt of the substance of Formula I is replaced by another anion under conditions which permit separation of the desired species, such as by precipitation from solution or extraction into a solvent, or elution from or retention on an ion exchange resin.

Pharmaceutically acceptable acids for the purposes of salt formation of the substances of Formula I include fumaric, sulfuric, phosphoric, hydrochloric, hydrobromic, hydroiodic, citric, acetic, benzoic, cinnamic, mandelic, phosphoric, nitric, mucic, isethionic, palmitic, heptanoic, and the like.

Additionally, compounds of Formula I also encompass all pharmaceutically acceptable solvates, hydrates being preferred solvates. The present invention also includes both geometrical isomers and optical isomers, e.g., mixtures of enantiomers as well as individual enantiomers and diastereomers, which arise as a consequence of structural asymmetry in certain compounds of the instant series. In general, (-)-enantiomers are preferred. Separation or stereospecific syntheses of the individual isomers is accomplished by application of various methods which are well known to practitioners in the art. Descriptive details of the synthesis of compounds of Formula I and of intermediates appears below.

One synthetic route to the indanyl piperazines and homopiperazines that avoids undesired indene byproducts, (V, in Method a, above ), gives higher yields under less critical conditions. This synthesis comprises a modification of the titanium(IV) isopropoxide reductive amination procedure described by R.J. Mattson, et al. [J. Organic Chemistry, 55, 2553 (1990)]. This technique uses an excess of piperazine or homopiperazine to give the desired indanyl piperazines or homopiperazines, I-i, in good yield. A preferred modification of this route (Method 2) to the compounds of formula I-i, however, uses the mono-formamide of piperazine or homopiperazine. The use of the formyl protecting group obviates the need for the large excess of the piperazine. The formyl protecting group is cleanly hydrolyzed during usual reaction workup with aqueous hydroxide. Both of these methods form the indanyl piperazine or homopiperazine product in one step from the indanone starting material. 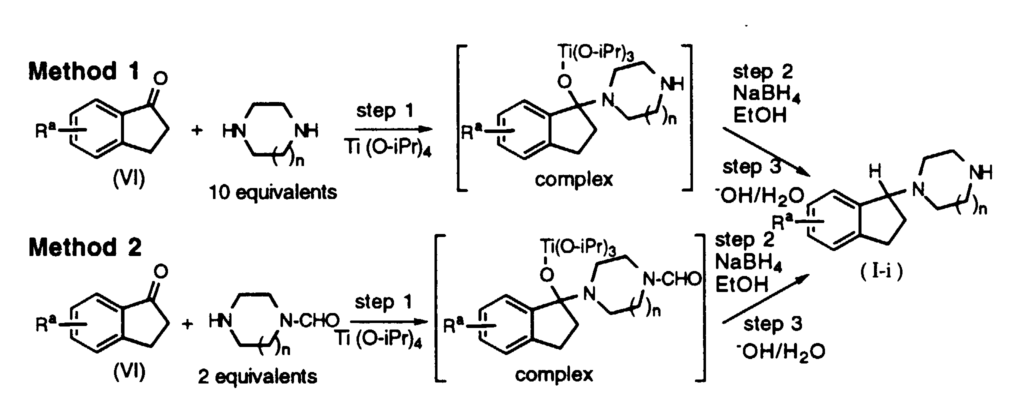 R^{a} is H, C₁₋₄ alkyl, C₁₋₉ alkoxy or halogen; n is 1 or 2.

One preferred method of making piperazines or homopiperazines involves the steps:
(1) condensing an indanone of formula VI 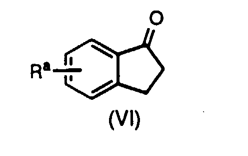 with a piperazine, homopiperazine, N-formyl piperazine, or N-formyl homopiperazine in the presence of titanium(IV) isopropoxide to form a titanium/indanone/piperazine complex;
(2) reducing the complex produced in step (1) with sodium borohydride, sodium cyanoborohydride in a suitable solvent;
(3) quenching the reaction using aqueous base; and
(4) recovering the formula I-i product.

The compounds of the present invention have affinity for human receptors of the endogenous pineal hormone, melatonin, as determined in a functional assay. The biological testing is described hereinbelow.

As has been discussed above, melatonin is involved in the regulation of a variety of biological rhythms and exerts its biological effects via interaction with specific receptors. There is evidence that administration of melatonin agonists is of clinical utility in the treatment of various conditions regulated by melatonin. Such conditions indude depression, jet-lag, work-shift syndrome, sleep disorders, glaucoma, some disorders associated with repreduction, cancer, immune disorders and neuroendocrine disorders.

The systemic administration and dosing regimen of compounds of Formula I can be done in a manner similar to that described for melatonin itself. The dosage and dosage regimen must be adjusted using sound professional judgment and taking into account such variables as the age, body weight, sex and physical condition of the recipient, the route of administration and the nature of the illness being treated.

Preferred classes of compounds of Formula I indude those in which R is methoxy, m and n are 1, Y is H, Z is O or S, and R¹ is C₁₋₄ alkyl, C₃₋₆ cydoalkyl, thienyl, furanyl, and NHR², with R² being straight or branched C₁₋ ₄ alkyl. Salts of these are also useful.

The preferred compounds include:
1-(Cyclopropylcarbonyl)-4-(6-methoxy-indan-1-yl)piperazine;
1-Butanoyl-4-(6-methoxy-indan-1-yl)piperazine;
1-(Cyclobutylcarbonyl)-4-(6-methoxy-indan-1-yl)piperazine;
4-(6-Methoxy-indan-1-yl)-1-(2-methyl-propanoyl)piperazine;
1-[(1-Methyl-ethenyl)carbonyl]-4-(6-methoxy-indan-1-yl)piperazine;
(-)-1-(Cyclopropylcarbonyl)-4-(6-methoxy-indan-1-yl)piperazine;
(-)-4-(6-Methoxy-indan-1-yl)-1-(2-methyl-propanoyl)piperazine;
(-)4-(6-Methoxy-indan-1-yl)-1-(2-methylthioacetyl)piperazine;
1-(Cydopropylcarbonyl)-4-(6-ethoxy-indan-1-yl)piperazine;
4-(6-Methoxy-indan-1-yl)-1-propanoylpiperazine;
1-(Cyclopentylcarbonyl)-4-(6-methoxy-indan-1-yl)piperazine;
1-(Ethenylcarbonyl)-4-(6-methoxy-indan-1-yl)piperazine;
4-(6-Methoxy-indan-1-yl)-1-(2-methyl-propanoyl)homopiperazine;
1-(Cyclopropylcarbonyl)-4-(6-methoxy-indan-1-yl)homopiperazine;
1-(2,2-dimethyl-propanoyl)-4-(6-methoxy-indan-1-yl)piperazine;
1-(Methoxyacetyl)-4-(6-methoxy-indan-1-yl)piperazine;
(-)-1-[(2,2-Dimethyl-ethenyl)carbonyl]-4-(6-methoxy-indan-1-yl)piperazine;
(-)-1-(Chloroacetyl)-4-(6-methoxy-indan-1-yl)piperazine;
4-(6-Methoxy-indan-1-yl)-1-(2-methyl-butanoyl)piperazine;
(-)-1-(2,2-Dimethyl-butanoyl)-4-(6-methoxy-indan-1-yl)piperazine;
(-)-4-(6-Methoxy-indan-1-yl)-1-(2-oxo-butanoyl)piperazine;
1-(Cyclopropylcarbonyl)-4-[6-(1-propyloxy)indan-1-yl]piperazine;
1-(Cyclopropylcarbonyl)-4-(5-fluoro-6-methoxy-indan-1-yl)piperazine;
4-(5-Fluoro-6-methoxy-indan-1-yl)-1-(2-methylpropanoyl )piperazine;
1-Acetyl-4-(6-methoxy-indan-1-yl)piperazine;
4-(6-Methoxy-indan-1-yl)-1-(2-methyl-propanoyl)piperazine;
1-(Cydopropylcarbonyl)4-(indan-1-yl)piperazine;
(-)-1-[(1-Methylcyclohexyl)carbonyl]-4-(6-methoxy-indan-1-yl)piperazine;
(-)-4-(6-Methoxy-indan-1-yl)-1-(10-undecenoyl)piperazine;
(-)-4-(6-Methoxy-indan-1-yl)-1-(4-pentenoyl)piperazine;
1-(Cyclopropylcarbonyl)-4-(6-nonyloxy-indan-1-yl)piperazine;
1-(Cydopropylcarbonyl)-4-(6-ethyl-indan-1-yl)piperazine;
4-(6-Ethyl-indan-1-yl)-1-(2-methylpropanoyl)piperazine;
4-(6-Methoxy-indan-1-yl)-1-[(2-thienyl)carbonyl]piperazine;
1-[(2-Furanyl)carbonyl]-4-(6-methoxy-indan-1-yl)piperazine;
(-)-4-(6-Methoxy-indan-1-yl)-1-[(2-thienyl)carbonyl]piperazine;
(-)-1-[(2-Furanyl)carbonyl]-4-(6-methoxy-indan-1-yl)piperazine;
(-)-4-(6-Methoxy-indan-1-yl)-1-[(3-methyl-furan-2-yl)carbonyl] piperazine;
(-)4-(6-Methoxy-indan-1-yl)-1-[(1-methyl-pyrrol-2-yl)carbonyl]piperazine;
(-)-4-(6-Methoxy-indan-1-yl)-1-[(1-methyl-thien-2-yl)carbonyl]piperazine;
(-)-4-(6-Methoxy-indan-1-yl)-1-[(3-thienyl)carbonyl]piperazine;
(-)-1-[(3-Chlororothien-2-yl)carbonyl]A-(6-methoxy-indan-1-yl)-1-piperazine;
(-)1-[(3-Bromothien-2-yl)carbonyl]-4-(6-Methoxy-indan-1-yl)piperazine;
(-)-1-(2-Hydroxybenzoyl)-4-(6-methoxy-indan-1-yl)piperazine;
(-)-1-[(3-Furanyl)carbonyl]-4-(6-methoxy-indan-1-yl)piperazine;
(-)-4-(6-Methoxy-indan-1-yl)-1-[(1H-pyrrol-2-yl)carbonyl]-piperazine;
(-)-4-(6-Methoxy-indan-1-yl)-1-[(1,2,3-thiadiazol-4-yl)carbonyl]-piperazine;
(-)-1-[(5-Bromo-furan-2-yl)carbonyl]-4-(6-methoxy-indan-1-yl)piperazine;
(-)-1-(Benzoyl)-4-(6-methoxy-indan-1-yl)piperazine;
(-)-1-[(1H-indol-3-yl)carbonyl]-4-(6-methoxy-indan-1-yl)piperazine;
(-)-1-[(4,5-Dibromo-thien-2-yl)carbonyl)
(-)-1-(2-Chlorobenzoyl)-4-(6-methoxy-indan-1-yl)piperazine;
(-)-1-[(5ehloro-thien-2-yl)carbonyl]-4-(6-methoxy-indan-1-yl)piperazine;
1-(Ethoxycarbonyl)-4-(6-methoxy-indan-1-yl)piperazine;
N-Cyclopropyl-4-(6-methoxy-indan-1-yl)piperazine-1-carboxamide;
N-Methyl-4-(6-methoxy-indan-1-yl)piperazine-1-carboxamide;
(-)-N-Ethyl-4-(6-methoxy-indan-1-yl)piperazine-1-carboxamide;
N-cyclopropyl-4-(6-methoxy-indan-1-yl)piperazine-1-carboxamide;
N-(n-Propyl)-4-(6-methoxy-indan-1-yl)piperazine-1-carboxamide;
N-Ethyl-4-(6-methoxy-indan-1-yl)piperazine-1-carboxamide;
N-Ethyl-4-(6-methoxy-indan-1-yl)homopiperazine-1-carboxamide;
N-(1-Methylethyl)-4-(6-methoxy-indan-1-yl)piperazine-1-carboxamide;
N,N-Dimethyl-4-(6-methoxy-indan-1-yl)piperazine-1-carboxamide;
N-Ethyl-4-(6-ethoxy-indan-1-yl)piperazine-1-carboxamide;
N-Ethyl-4-(6-propyloxy-indan-1-yl)piperazine-1-carboxamide;
N-Ethyl-4-(6-nonyloxy-indan-1-yl)piperazine-1-carboxamide;
N-Ethyl-4-(5-fluoro-6-methoxy-indan-1-yl)piperazine-1-carboxamide;
N-Methyl-4-(5-fluoro-6-methoxy-indan-1-yl)piperazine-1-carboxamide;
1-Acetyl-4-(7-methoxy-tetralin-1-yl)piperazine;
1-(1-Butanoyl)-4-(7-methoxy-tetralin-1-yl)piperazine;
1-(Cyclopropylcarbonyl)-4-(7-methoxy-tetralin-1-yl)piperazine;
N-Methyl-4-(7-methoxy-tetralin-1-yl)piperazine-1-carboxamide; and
N-Ethyl-4-(7-methoxy-tetralin-1-yl)piperazine-1-carboxamide.

Compounds of Formula I and their salts can be prepared using the processes shown in the following scheme(s): **SCHEME 1:** Preferred Synthesis of Compounds of Formula I 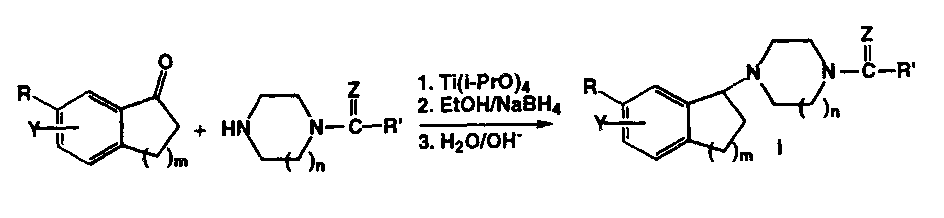 **SCHEME 2:** Synthesis of Piperazine Intermediates 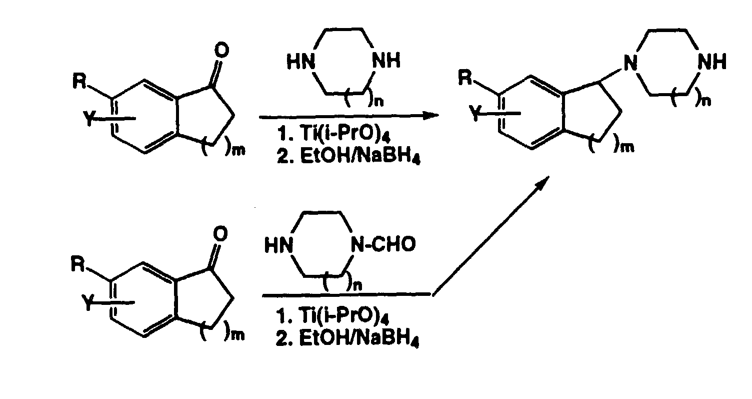 **SCHEME 3:** Synthesis of Piperazine Derivatives of Formula I

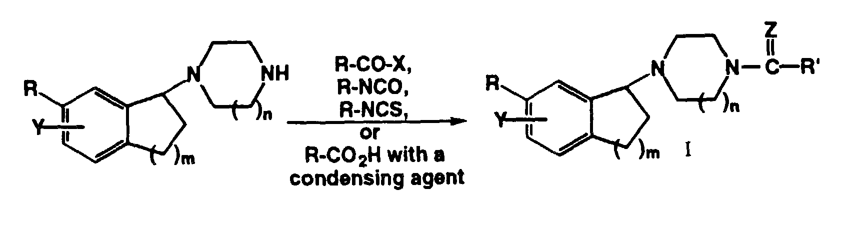

The most preferred route (Scheme 1) to the Compounds of Formula I comprises a modification of the titanium(IV) isopropoxide reductive amination procedure described by R.J. Mattson, et al. [J. Organic Chemistry, 55, 2553 (1990)]. In this method (Scheme 1) an acyl-piperazine is reductively coupled with the substituted ketone to give the products, **I**, directly in one step.

Alternatively, the substituted ketone can be condensed (Scheme 2) with either piperazine or more preferably with 1-formyl-piperazine to give the indanyl-piperazine intermediate. The 1-formyl-piperazine negates the need for the large excess of piperazine and is cleanly hydrolyzed during the work up to give the indanyl-piperazine intermediate. This intermediate can then be acylated (Scheme 3) using standard methods to give the products of Formula **I**.

These processes may be adapted in order to produce other compounds embraced by the invention, but not specifically disclosed herein. Variations of these methods to produce compounds via different, but conventional, routes will be evident to one skilled in the art. Representative examples are set out in "Description of Specific Embodiments" section, below.

The compounds of Formula **I** and their salts are melatonergic agents. Their melatonergic activity has been demonstrated via receptor binding studies using human melatonin receptors as set out in Example 21.

The compounds of the invention may be administered to patients in need of melatonergic treatment in a variety of ways. Thus oral, transdermal, subcutaneous, intravenous, intramuscular, rectal, buccal, intranasal and ocular routes can be used.

One or more of the compounds of the invention is mixed with pharmaceutically suitable amounts of one more conventional pharmaceutical excipients to produce a formulation to be administered by the desired route. Generally, such formulations will contain one or several carriers or diluents. Useful carriers include solids, semi-solids, and liquids which have miscibility, or other compatability, with the active agents(s) so the they can efficiently deliver same to a patient or other host.

Suitable carriers include lactose, dextrose, sucrose, sorbitol, mannitol, starches, gum acacia, calcium phosphate, alginates, tragacanth, gelatin, calcium silicate, microcrystalline cellulose, polyvinyl pyrrolidone, cellulose, water, syrup, methyl cellulose, methyl- and propyl-hydroxybenzoates, talc, magnesium stearate, mineral oil and the like. Mixtures are operable.

Other useful excipients indude, lubricants, wetting agents, gellants, emulsifiers, preservatives, colorants, perfumes, flavor enhancers, drying agents and the like Mixtures can be employed.

Generally, compositions which include the compounds of the invention will contain from about 0.10 to about 10% by weight of active compound(s) and 99.9 to 90 % by weight, or other suitable amounts, of excipients(s).

Dosage levels will be dictated by the patient's needs and by the medical judgment of the treating physician. Generally, however, dosages of about 0.1 to about 100 mg per day are useful to treat sleep disorders or other disorders related to circadian rhythm.

While human patients are preferred, the compounds of the invention may be used to treat other subjects, i.e., animals, preferably mammals.

The compounds of the invention, their preparation and their biological properties will be more clearly understood upon consideration of the following examples. The examples are illustrative only and are not intended to limit the scope the invention.

In these examples, temperatures are expressed in degrees Celsius and melting points are uncorrected. The elemental analysis results are reported as percent by weight. All percentages are, unless less designated otherwise, weight percent based on total composition weight.

### Examples:

### Preparation of Intermediates

The following examples illustrate the process of making piperazine and homopiperazine intermediates.

Example 1

(6-Methoxyindan-1-yl)piperazine

### Method A

Step 1: An intimate mixture of 6-methoxy-1-indanone (10 g, 62 mmol), piperazine (53 g, 620 mmol) and titanium(IV) isopropoxide (17.6 g, 124 mmol) was heated on the steam bath for 10 minutes (step 1). The IR spectrum of the mixture showed no carbonyl absorption.

Step 2: The material was dissolved in ethanol and sodium borohydride added (2.5 g, 62 mmol). After stirring for 1 hr the solution was heated to reflux.

Step 3: When a solution had occurred 15% NaOH solution (50ml) was added. The insoluble material was filtered and discarded. The solution was concentrated *in vacua* and the residue dissolved in ether. The solution was washed with water and 1N HCl solution. The acid washes were made basic and the mixture was extracted with methylene chloride to give the product, which was converted to the HCl salt in absolute ethanol [beige solid, 13.3 g, 80%, mp: 150-152 °C (HCl salt)]. Calcd for C₁₄H₂₀N₂O•HCl•0.5H₂O: C, 60.52; H, 7.98; N, 10.08. Found: C, 60.59; H, 7.65; N, 10.11.

### Method B

Step 1: A mixture of 6-methoxy-indan-1-one (1.6 g, 10 mmol), 1-formyl piperazine (2.3 g, 20 mmol), and titanium(IV) isopropoxide (7 ml, 21 mmol) was heated gently on a steam bath for 10 min. The IR spectrum of the mixture then showed no ketone absorption.

Step 2: The mixture was dissolved in absolute ethanol (20 ml) and sodium borohydride (1.6 g, 40 mmol) was added. The reaction mixture was heated gently on a steam bath for 10 min and then stirred for 16 hr.

Step 3: The reaction was then heated to give a dear solution and then quenched with 15% sodium hydroxide (4 ml) to produce a white precipitate. The mixture was stirred for 1 hr and then filtered. The precipitate was washed thoroughly with ethanol The combined filtrates were concentrated in vacuo and the residue partitioned between diethyl ether and 1 N HCl. The aqueous layer was made basic with with sodium carbonate and then extracted with methylene chloride. The extracts were dried over sodium sulfate and concentrated *in vacuo* to give the product (1.7 g, 73.3 %).

### Example 2

### 1-(Indan-1-yl)piperazine

Piperazine (17.2 g, 0.2 mol), indan-1-one (2.6 g, 20 mmol), titanium(IV) isopropoxide (13.3 ml, 40 mmol) and sodium borohydride (3.6 g, 95 mmol) were reacted by Method A to give the product (1.5 g, 37.1%). The crude product was used without purification.

### Example 3

### 1-(6-Ethoxyindan-1-yl)piperazine

Piperazine (19.8 g, 0.23 mol), 6-ethoxyindan-1-one (4.00 g, 23 mmol), titanium(IV) isopropoxide (15.3 ml, 46 mmol) and sodium borohydride (2.7 g, 71 mmol) were reacted by Method A to give the product which was converted to the fumurate salt (beige solid, 5.7 g, 68.5%, mp: 142-147°C). Calcd for C₁₅H₂₂N₂O• C₄H₄O₄: C, 62.97%; H, 7.23%; N, 7.73%. Found: C. 62.88%; H, 7.22%; N, 7.48%.

### Example 4

### 1-[6-(1-Propyloxy)indan-1-yl]piperazine

Piperazine (20.6 g, 0.24 mol), 6-(1-propoxy)indan-1-one (4.6 g, 24 mmol), titanium(IV) isopropoxide (16ml, 48 mmol) and sodium borohydride (2.8 g, 74 mmol) were reacted by Method A to give the product which was converted to the fumarate salt (beige solid, 5.0 g, 55.4%, mp: 157-159°C). Calcd for C₁₆H₂₄N₂O •C₄H₄O₄•0.3 H2O: C, 62.91%; H, 7.55%; N, 7.34%. Found: C, 62.70%; H, 7.45%; N, 7.07%.

### Example 5

### 1-(6-methoxy-indan-1-yl)homopiperazine

Homopiperazine (30.6 g, 0.306 mol), 6-methoxy-indan-1-one (4.95 g, 30.56 mmol), titanium(IV) isopropoxide (17.9 ml, 60 mmol) and sodium borohydride (2.7 g, 71 mmol) were reacted by Method A to give the product (5.3 g, 70.5%). The crude product was used without purification.

### Example 6

### (6-Ethylindan-1-yl)piperazine

### Step 1. 4-Ethyl cinnamic acid

A solution of 4-ethylbenzaldehyde (26.0 g, 0.2 mol), malonic acid (41.6 g, 0.4 mol), pyrrolidine (3 ml), and pyridine (80 ml) was heated in an 85°C oil bath for 16 hr. The reaction mixture was poured over crushed ice (800 ml), and then made acidic with 12 N HCl (100 ml). The white precipitate was filtered, suspended in 1 N HCl, and filtered again. The white precipitate was washed with water and air dried to give 4-ethyl cinnamic acid (34.0 g, 96.6).

### Step 2. 3-(4-Ethylphenyl)propionic acid.

An ethanol (250 ml) solution of 4-ethyl cinnamic acid (34.0 g, 0.191 mol) was hydrogenated at 60 psi over 10% Pd/C (2 g) for 2 hr. The mixture was filtered and concentrated *in vacuo* to give 3-(4-ethylphenyl)propionic acid as a white solid (34 g, 100%).

### Step 3. 6-Ethyl-1-indanone.

A mixture of 3-(4-ethylphenyl)propionic acid (34 g, 0.191 mol) and thionyl chloride (70 ml), and CH₂Cl₂ (100 ml) was heated to reflux for 30 min. The resulting solution was concentrated *in vacuo* to a light brown oil. This oil was added slowly to an ice bath cooled mixture of AlCl₃ (33.1 g, 0.248 mole) in CH₂Cl₂ (75 ml). The mixture was stirred for 15 min and then heated to reflux for 45 min. The mixture was cooled and poured over crushed ice (200 ml) and 12 N HCl (100 ml). The CH₂Cl₂ layer was separated, washed with 3N HCl, saturated Na₂CO₃, water, and brine. The CH₂Cl₂ layer was concentrated in vacuo and Kügelrohr distilled to a dear oil (27.4 g, 89%). ¹H-NMR (CDCl₃, 300 MHz)δ 1.23 (t, 3H, J=7.5 Hz), 2.65-2.72 (m, 4H), 3.07 (t, 2H, J=5.9 Hz), 7.36 (d, 1H, J=8.1 Hz), 7.41 (d, 1H, J=8.1 Hz), 7.57 (s, 1H).

### Step 4. (6-Ethylindan-1-yl)piperazine.

This compound, prepared from 6-ethyl-1-indanone, piperazine and titanium isopropoxide as described in method B above, was isolated as the fumarate salt (50 %, mp 151-153 °C).

Calc'd for C₁₅H₂₂N₂•C₄H₄O₄: C, 65.87%; H, 7.57%; N, 8.09%. Found: C, 65.59%; H, 7.50%; N, 8.00%.

### Example 7

### 1-(5-fluoro-6-methoxy-indan-1-yl)piperazine

3-Fluoro-4-methoxybenzaldehyde was converted to 5-fluoro-6-methoxy-1-indanone by method 2, steps 1-4 above, (92%, mp 149-151° C). Anal. Calc'd for C₁₀H₉FO₂: C, 66.66%; H, 5.03%. Found: C, 66.65%; H, 4.96%.

1-Formyl-piperazine (2.1 g, 20 mmol), 5-fluoro-6-methoxy-indan-1-one (1.8 g, 10 mmol), titanium(IV) isopropoxide (5 ml, 15 mmol), and sodium borohydride (1.2 g, 30 mmol) were reacted by Method B to give the product (2.2 g, 88%, mp 163-166 °C). Calcd for C₁₄H₁₉FN₂O • C₄H₄O₄ • 0.1 H₂O: C, 56.23%; H, 6.56%; N, 7.28%. Found: C, 56.65%; H,6.41%; N, 6.98%.

### Example 8

### 1-[6-(1-nonyloxy)indan-1-yl]piperazine

### Step 1. 6-(1-Nonyloxy)-1-indanone.

A solution of 6-hydroxy-1-indanone (0.9 g, 6 mmol), NaOH (6 mL 1 N sol'n, 6 mmol) and 1-bromononane (1.4g, 6.6 mmol) in ethanol (50 mL) was heated at reflux for 14 h and the solution concentrated in vacuo. The residue was dissolved in ether and the solution washed with 1 N NaOH solution. The ether layer was dried and purified by chromatography on silica eluting with ethyl acetate-hexane (1:9) to give the product, (67%, mp: 41-42° C). C₁₈H₂₆O₂: C, 78.79%; H, 9.55%. Found: C, 78.69%; H, 9.44%.

### Step 2. 1-[6-(1-Nonyloxy)indan-1-yl]piperazine.

Piperazine (4.4 g, 51 mmol), 6-(1-nonyloxy)indan-1-one (1.4 g, 5.1 mmol), titanium(IV) isopropoxide (2.5 ml. 7.5 mmol) and sodium borohydride (1.8 g, 47 mmol) were reacted by Method B to give the product (1.6 g, 91.2%, mp: 138-144°C). Calcd for C₂₂H₃₆N₂O•C₄H₄O₄•0.4H₂O: C, 66.75%; H, 8.79%; N, 5.99%. Found: C, 66.76%; H, 8.71%; N, 5.90%.

### Example 9

### (7-Methoxytetralin-1-yl)piperazine

This compound, prepared from 7-methoxy-tetralin-1-one, piperazine, and titanium(IV) isopropoxide as described in method B above, was isolated as the fumarate salt (58%, mp: 185-186 °C).
Calc'd for C₁₅H₂₂N₂O•C₄H₄O₄: C, 62.97%; H, 7.23%; N, 7.73%. Found: C, 63.02%; H, 7.31; N, 7.63%.

### Example 10 :

### Resolution of (6-methoxy-indan-1-yl)piperazine.

A mixture of (6-methoxy-indan-1-yl)piperazine (2.3 g, 10 mmol) and (1S)-(+)-10-camphorsulfonic acid (2.3 g, 10 mmol) was recrystallized from ethanol-water until the crystal attained a constant melting point yielding (-)-(6-methoxy-indan-1-yl)piperazine (1S)-10-camphorsulfonic acid salt (22%, mp: 234.5-234 °C, [α]²⁵D -36.3°).
Calc'd for C₁₄H₂₀N₂O₂•C₁₀H₁₀O₄S: C, 62.04%; H, 7.81%; N, 6.03%. Found: C, 62.01%; H, 7.96; N, 5.97%.

A sample (0.2 g, 0.43 mmol) of the above salt was suspended in water and the mixture made basic with 1N NaOH. The basic solution was extracted with CH2Cl₂. The extracts were then dried and concentrated *in vacuo.* NMR analysis of this free base using a chiral shift solution showed none of the enantiomeric compound. A solution of fumaric acid (49 mg) in methanol was added, the solution concentrated *in vacuo* and the residue crystallized to give the fumarate salt, (mp: 170-172 °C, [α]²⁵D -72.3°)
Calc'd for C₁₄H₂₀N₂O₂•C₄H₄O₄:C, 62.05%; H, 6.94%; N, 8.04%. Found: C, 62.08%; H, 6.61%; N, 8.26%.

### General Procedure for Preparation of Amides, Urethanes, and tertiary-Ureas of Formula I:

### Example 11

### 1-Cyclopropylcarbonyl-4-(6-methoxy-indan-1-yl)piperazine

A mixture of (6-methoxy-indan-1-yl)piperazine (3.0 g, 15.5 mmol), cyclopropane-carbonyl chloride (1.6 g, 15.5 mmol), and excess powdered potassium carbonate was stirred for 6 hr. The insoluble material was removed and the solution concentrated *in vacuo.* The residue was dissolved in ether and washed with 1N HCl. The acid washes were made basic with NaOH solution and the mixture was extracted with methylene chloride. The extracts were dried and concentrated *in vacuo.* The residue was converted to the hydrochloride salt with ethereal HCl to give a solid (75%, mp: 187-188 °C). Calc'd for C₁₈H₂₄N₂O₂•HCl: C, 64.18%; H, 7.48%,-N, 8.32%. Found: C, 63.96%; H, 7.18%; N, 8.18%.

### Example 12

### 1-Acetyl-4-(7-methoxy-tetralin-1-yl)piperazine

This compound was prepared from (7-methoxytetralin-1-yl)piperazine and acetyl chloride by the method described in Example 11 above (pale yellow oil, 58%).
Calc'd for C₁₇H₂₄N₂O₂: C, 69.93%; H, 8.43%; N, 9.60%. Found: C, 69.94%; H, 8.18%; N, 9.45%.

Table 1 lists other compounds of Formula I prepared by the above method.

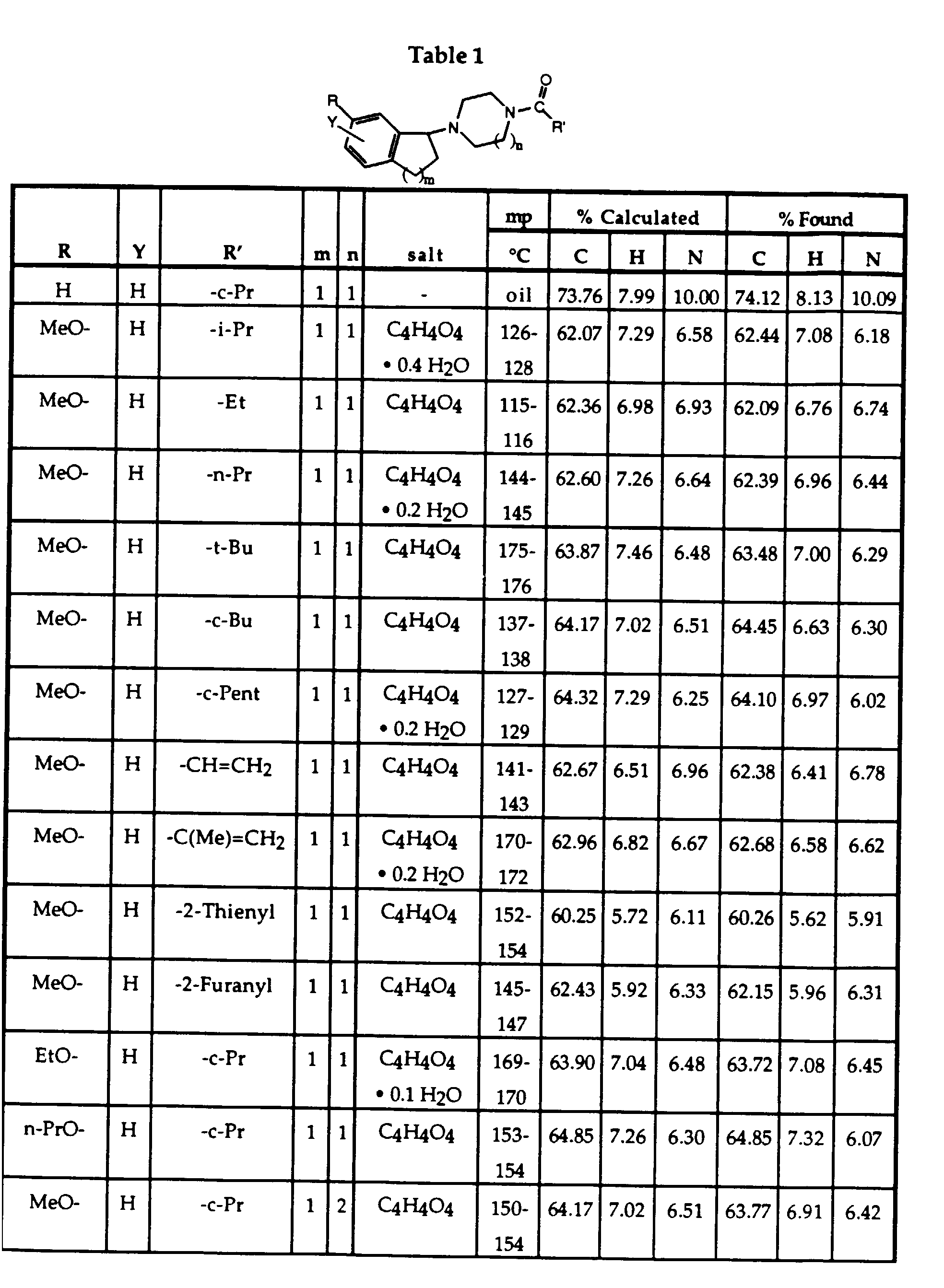

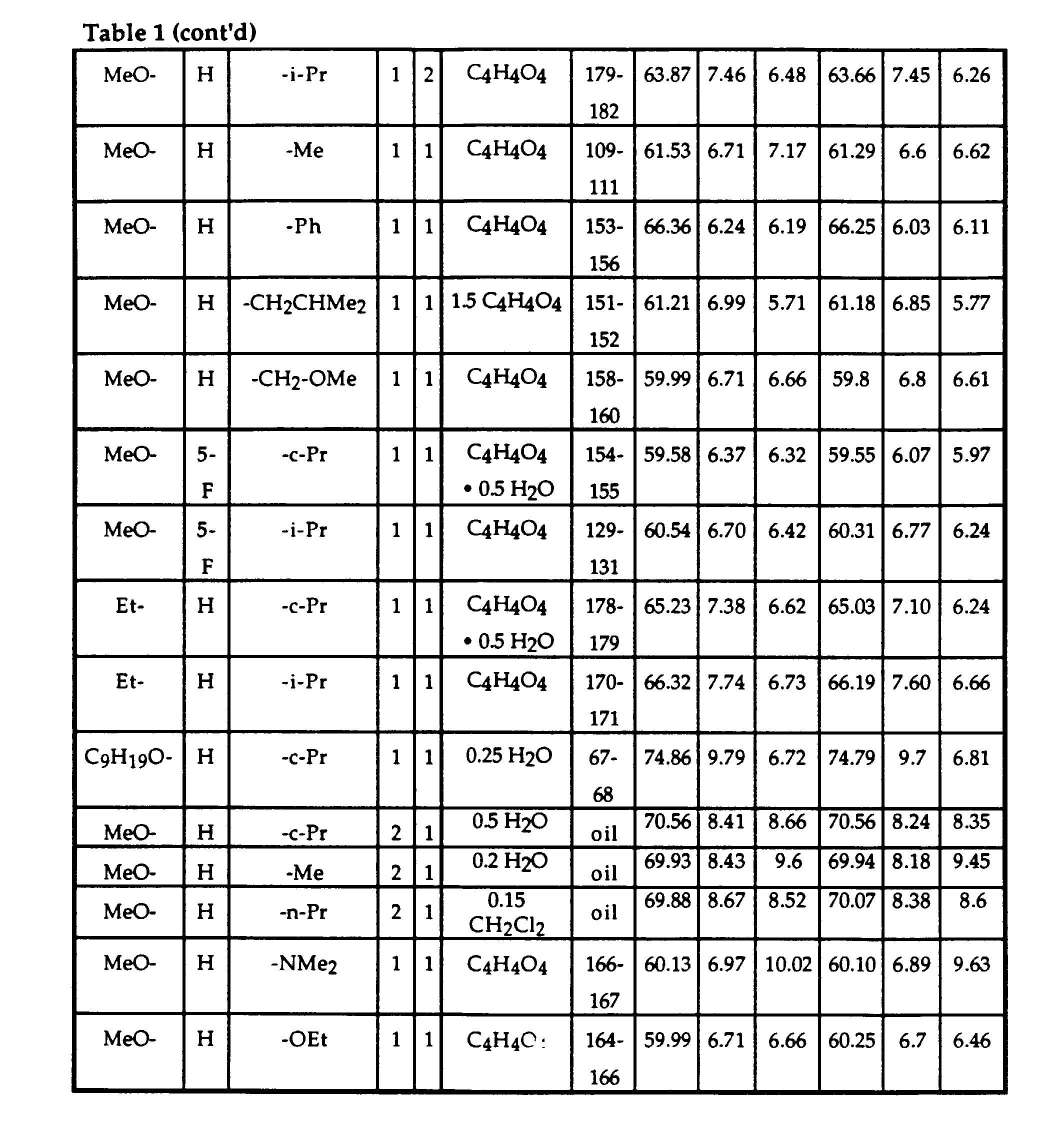

### General Procedure for Preparation of Secondary Ureas of Formula I:

### Example 13

### N-Methyl-4-(6-methoxy-indan-1-yl)-1-piperazinecarboxamide

Methyl isocyanate (0.25 g, 3.4 mmol) was added to a solution of (6-methoxy-indan-1-yl)piperazine (0.8 g, 3.4 mmol) in CH₂Cl₂. After stirring for 2 hr, the solution was washed with 1N HCl. The acid washes were made basic with NaOH solution and the mixture extracted with methylene chloride. The extracts were dried and concentrated *in vacuo.* The fumarate was prepared in methanol to give a solid, (85%, mp: 172-174 °C).
Calc'd for C₁₆H₂₃N₂O₂•C₄H₄O₄: C, 59.25%; H, 6.71%; N, 10.36%. Found: C, 58.92%; H, 6.95%; N, 9.96%

### Example 14

### N-Ethyl-4-(7-methoxy-tetralin-1-yl)-1-piperazinecarboxamide

This compound was prepared from ethyl isocyanate and 7-methoxytetralin-1-yl)piperazine by the method described in Example 13 above (74%, mp: 141-143 °C).

Table 2 lists other compounds of Formula I prepared by this method.

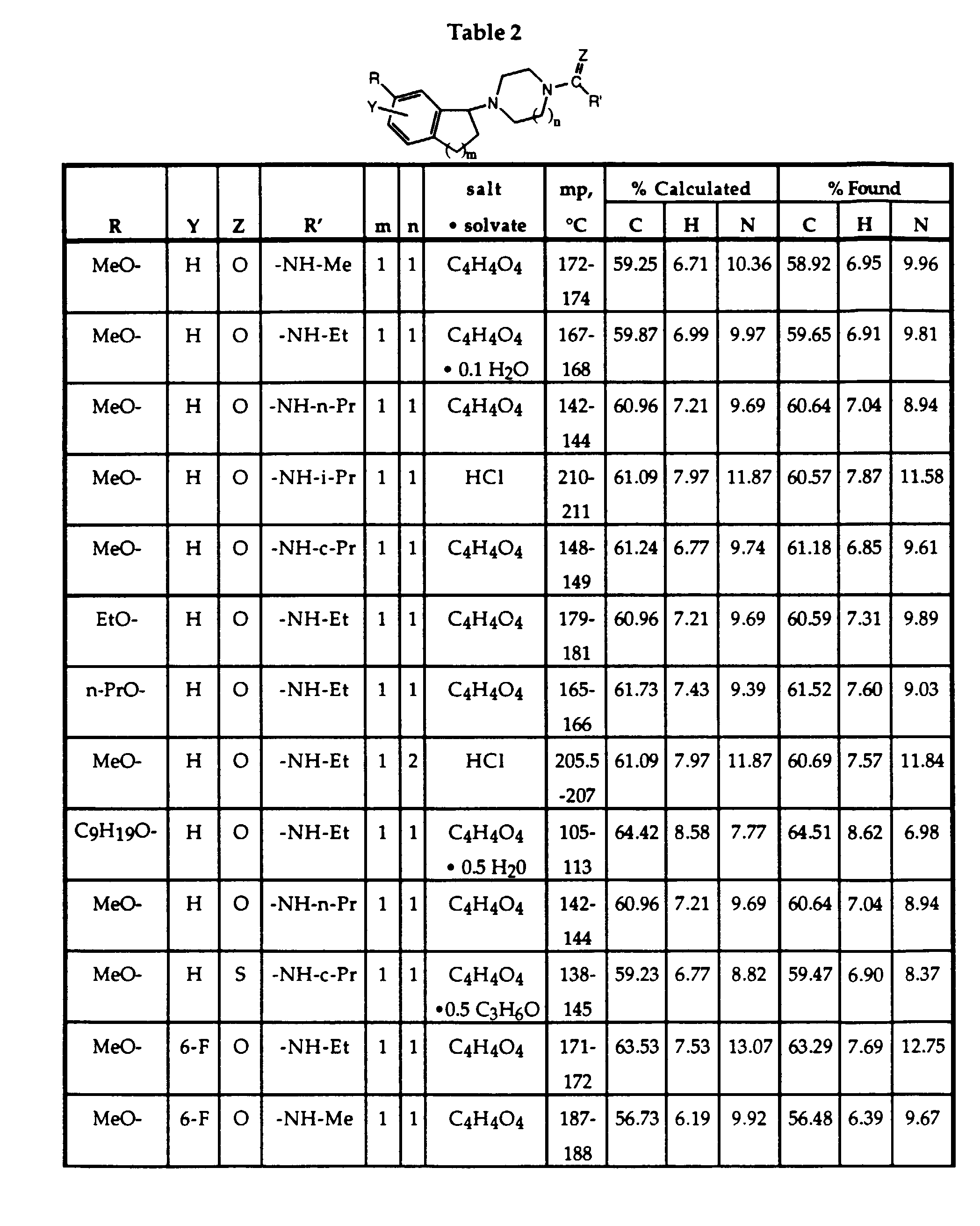

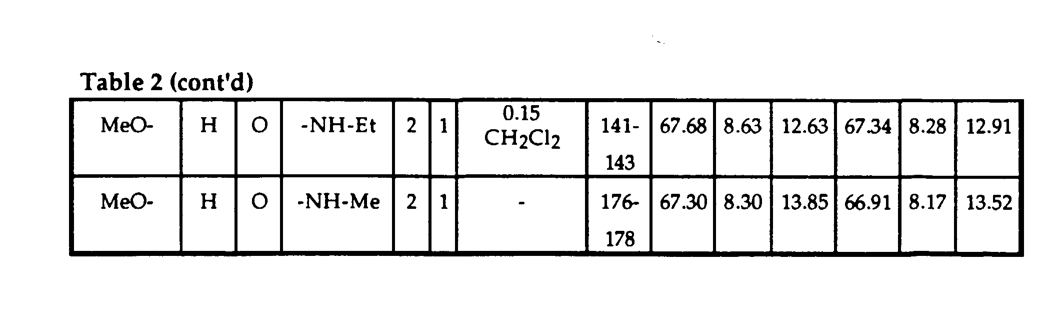

### Preferred Procedure for Preparation of Compounds of Formula I:

### Example 15

### 1-Cyclopropylcarbonyl-4-(6-methoxy-indan-1-yl)piperazine

An intimate mixture of 6-methoxy-1-indanone (1.6 g, 10 mmol), 1-cyclopropanecarbonylpiperazine (1.5 g, 10 mmol) and titanium(IV) isopropoxide (4 mL, 12 mmol) was heated on the steam bath for 10 minutes. Additional titanium isopropoxide (1 mL, 3 mmol) was added and the mixture stirred for 20 hr. The material was dissolved in ethanol and sodium borohydride added (0.9 g, 22 mmol). After stirring for 1 hr the solution was heated to reflux and more sodium borohydride (0.9 g, 22 mmol) added. When solution had occurred 15% NaOH solution (50 mL) was added. The insoluble material was removed and discarded. The solution was concentrated *in vacuo* and the residue mixed with ether. The mixture was washed with water and 1N HCl solution. The acid washes were made basic and the mixture was extracted with ether to give the product as an oil which was converted to the fumarate salt and crystallized from acetone to give the salt (1.8 g).

### Example 16

### 1-(2-Methylpropionyl)-4-(6-methoxy-indan-1-yl)piperazine

The title compound was prepared by the above procedure using 6-methoxy-1-indanone (3.2 g, 20 mmol), 1-(2-methylpropionyl)piperazine (3.0 g, 20 mmol), titanium(IV) isopropoxide (8 mL, 24 mmol) and sodium borohydride (2.7 g, 67 mmol) to give 2.2 g of product as the fumarate salt.

### Example 17

### 1-(2-Thienylcarbonyl)-4-(6-methoxy-indan-1-yl)piperazine

The title compound was prepared by the above procedure using 6-methoxy-1-indanone (2.6 g, 13.2 mmol), 1-(2-thienylcarbonyl)piperazine (2.1 g, 13.2 mol), titanium(IV) isopropoxide (5 mL, 15 mmol) and sodium borohydride (2.7 g, 67 mmol) to give 1.5 g of product as the fumarate salt.

### Example 18

### N-Ethyl-4-(6-methoxy-indan-1-yl)-1-piperazinecarboxamide

The title compound was prepared by the above procedure using 6-methoxy-1-indanone (2.4 g, 15 mmol), N-ethyl-1-piperazinecarboxamide (2.4 g, 15 mol), titanium(IV) isopropoxide (6 mL, 18 mmol) and sodium borohydride (2.7 g, 67 mmol) to give 2.8 g of product as the fumarate salt.

### General Procedure for Preparation of Chiral Amides and Ureas:

### Example 19

The appropriate chiral salt was converted to the free base as described above for preparation of fumarate salts. The CH₂Cl₂ solution was then reacted either with the appropriate isocyanate or acid chloride and potassium carbonate as described in the previous general procedures.

Table 3 lists chiral derivatives prepared by these methods.

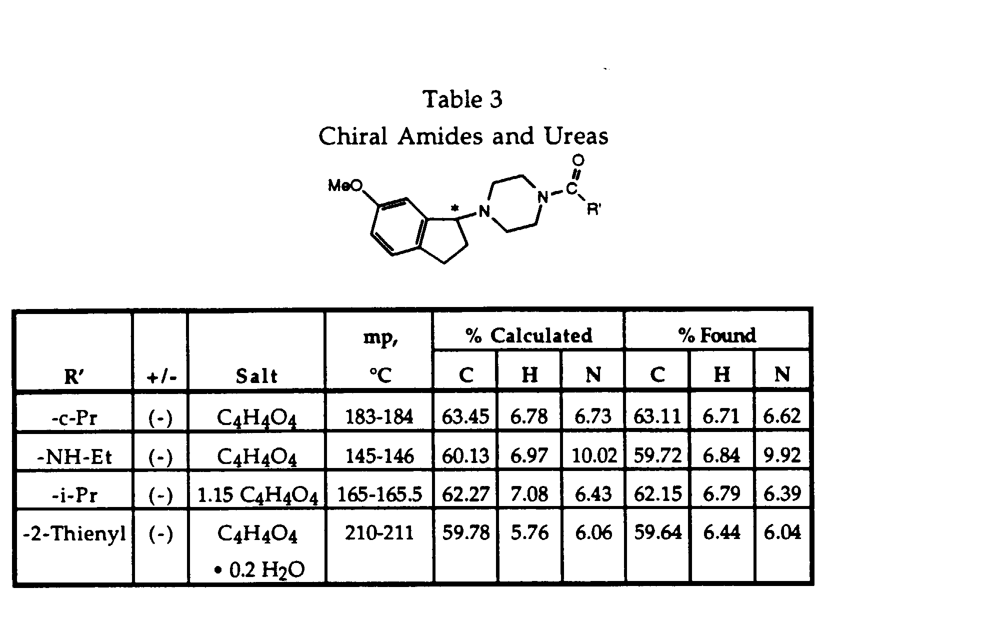

### Example 20

### 1-[(2-Furanyl)carbonyl]-4-(6-methoxy-indan-1-yl)piperazine

A solution of (-)-(6-methoxy-indan-1-yl)piperazine (28 mg, 0.12 mmol), 3-furoic acid (58 mg, 0.52 mmol), and 1-hydroxybenzotriazole (17 mg, 0.126 mmole), and 1,3-diisopropylcarbodiimide (16 mg, 0.126 mmol) in CH₂Cl₂ (2 ml) and DMF (2 ml), was shaken for for 5 min and allowed to stand for 18 hr. The reaction mixture was filtered through an SCX Bondesil® (Varian # 1221-3039, 1 g) column. The column was washed with methanol (20 ml) and 0.1 N NH₄OH in methanol (2 ml). The product was then eluted from the column using 1 N NH₄OH in methanol (5 ml). This last fraction was concentrated *in vacuo* to give the product (36.8 mg, 94%, ES-MS: 327 (MH+).

Table 4 lists other compounds of Formula I prepared by the above method from (-)-(6-methoxy-indan-1-yl)piperazine.

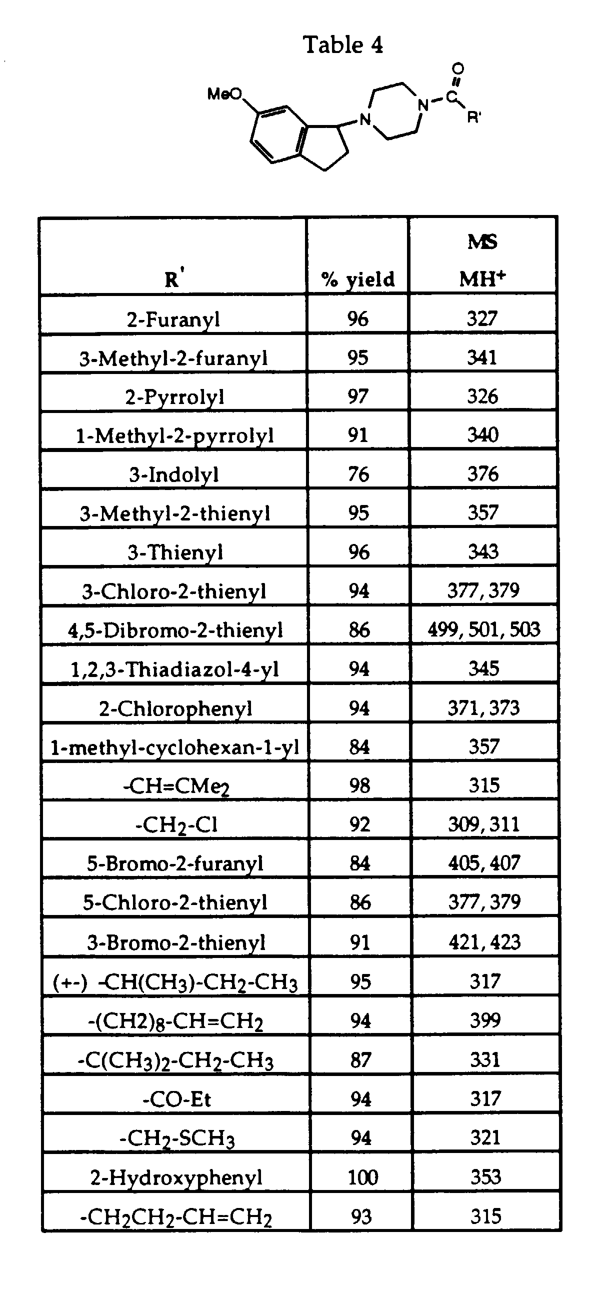

### Example 21

### Measurement of Binding to Melatonergic Receptors

The melatonergic binding affinities of various compounds of Formula I were determined by the method of Reppert, S. M., Weaver, D. R., and Ebisawa, R. *(Neuron,* Volume 13, 1177-1185, 1994). The assays were incubated at 37 °C for 1 hour, and the reaction was terminated by filtration. The filters were washed with wash buffer. Compounds with IC₅₀ affinity values at or below 250 nM are termed active. The reagents, membrane and other techniques used in the melatonergic binding assays are more fully described below:
1. Reagents:
   (a) 50 mM Tris buffer containing 12.5 mM MgCl₂, and 2mM EDTA, pH 7.4 at 37 °C.
   (b) Wash buffer: 20 mM Tris base containing 2 mM MgCl₂. pH 7.4 at room temperature.
   (c) Melatonin (10⁻⁵ M final concentration).
   (d) 2-[¹²⁵I]-Iodomelatonin, 200 pM final concentration Source: NEN
2. Membrane preparation: NIH 3T3 cells stably transfected with the human ML₁B receptor were obtained from S.M. Reppert and maintained. Cells were pelleted when confluent. The supernatant was discarded and the pellets frozen. For preparing membrane homogenates, the pellets are thawed on ice and resuspended in TME buffer, Tris base, MgCl₂, EDTA (pH 7.4 at 37 °C ), supplemented with aprotinin, leupeptin, and phenylmethlysulfonylfluoride. The cells were then homogenized and centrifuged. The resulting pellet was resuspended with a Dounce homogenizer in TME and frozen. At assay, a small aliquot was thawed on ice and resuspended in TME buffer.
Some compounds of Formula I having IC₅₀ value for melatonin binding of 250 nM or less are listed in Table 5. These compounds are considered active. The known melatonin antagonist, luzindole, is listed in Table 5 for comparison.

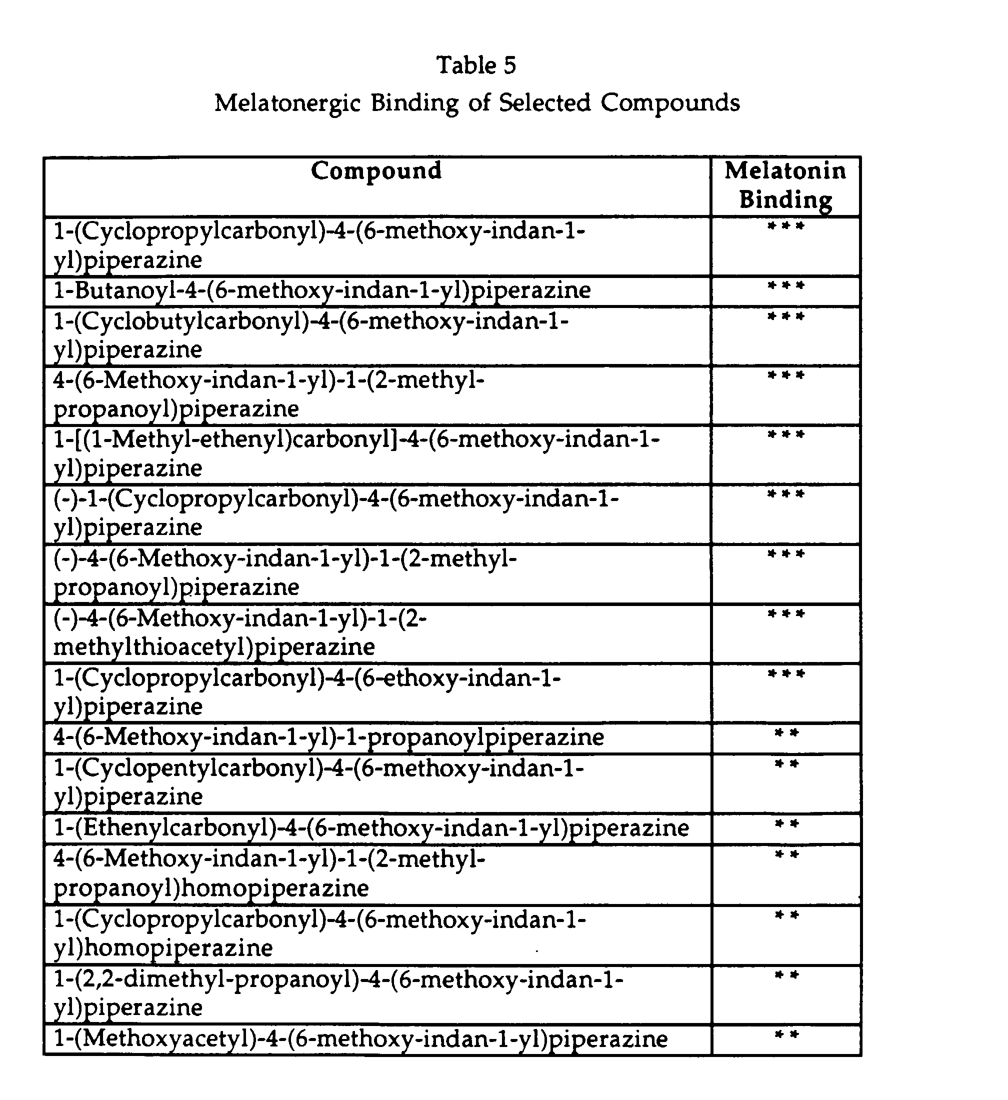

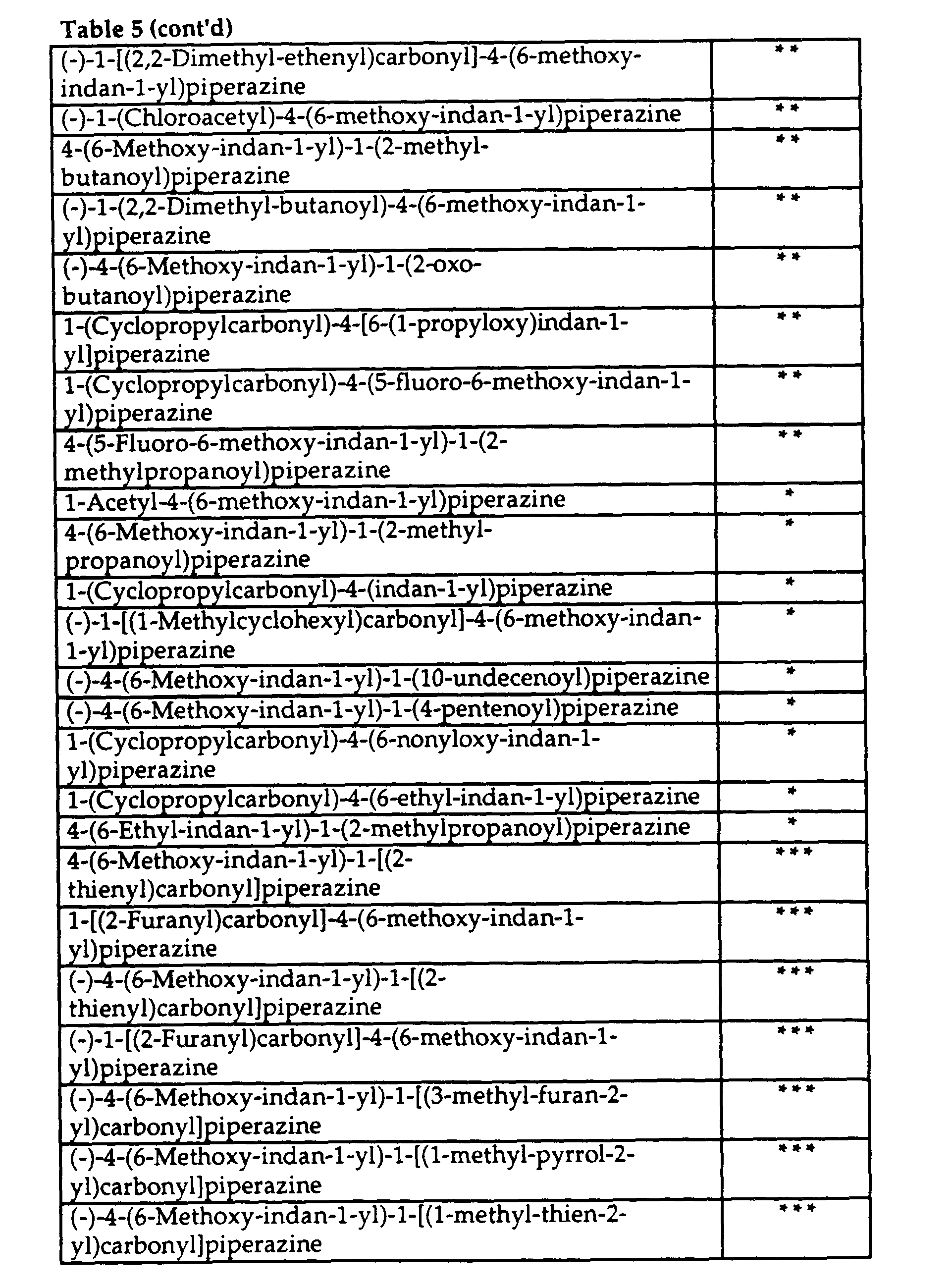

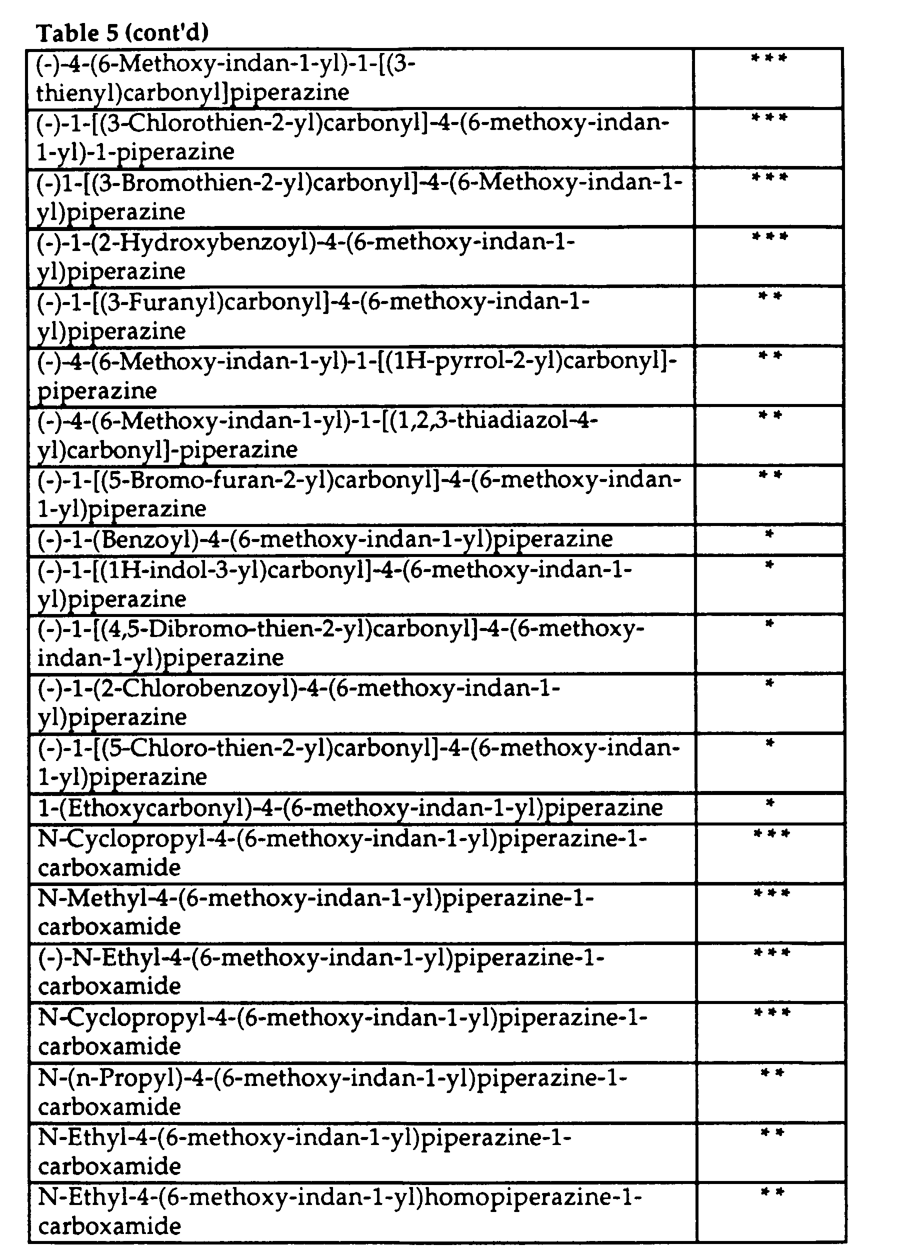

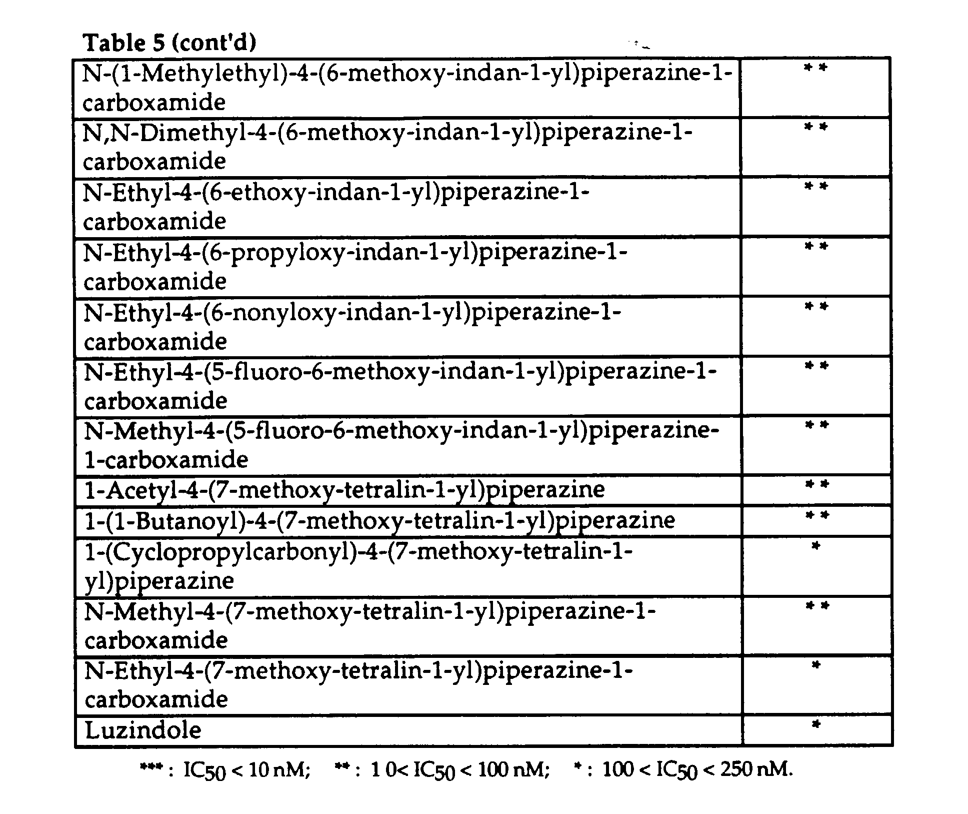

Reasonable variations, such as those which would occur to a skilled artisan, can be made herein without departing from the scope of the invention.

## Claims

1. A compound of Formula I: 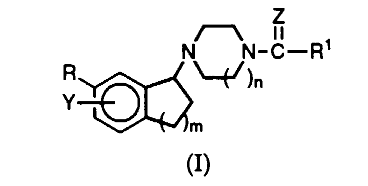 wherein:
R is H, C₁₋₄ alkyl, or C₁₋₉ alkoxy;
m is 1 or 2;
n is 1 or 2;
Y is H, C₁₋₄ alkoxy, or halogen;
Z is O or S; and
R¹ is C₁₋₆ alkyl (straight or branched), C₁₋₆ haloalkyl, C₁₋₄ alkoxy, C₁₋₄ thioalkoxy, C₁₋₄ thioalkoxy substituted C₁₋₄ alkyl, C₁₋₆ alkoxy substituted C₁₋₄ alkyl, C₂₋₂₀ alkenyl (straight or branched), C₃₋₆ cycloalkyl, phenyl, thienyl, pyrrolyl, furanyl, thiadiazolyl, indolyl, or NR²R³ wherein R² and R³ are independently hydrogen, C₁₋₃ alkyl or C₃₋₆ cycloalkyl.

2. The compound of claim 1 wherein R is methoxy, m is 1, n is and Z is O.

3. The compound of claim 2 wherein R¹ is C₃₋₅ cycloalkyl, thienyl, furanyl, pyrrolyl, phenyl or thioalkoxyalkyl.

4. The compound of claim 3 selected from the group consisting of:
(-)-4-(6-Methoxy-indan-1-yl)-1-[(2-thienyl)carbonyl]piperazine;
1-(Cyclopropylcarbonyl)-4-(6-methoxy-indan-1-yl)piperazine;
(-)-1-(Cyclopropylcarbonyl)-4-(6-methoxy-indan-1-yl)piperazine;
1-(Cyclopentylcarbonyl)-4-(6-methoxy-indan-1-yl)piperazine;
1-[(2-Furanyl)carbonyl]-4-(6-methoxy-indan-1-yl)piperazine;
4-(6-Methoxy-indan-1-yl)-1-[(2-thienyl)carbonyl]piperazine
(-)-1-[(3-Chlorothien-2-yl)carbonyl]-4-( an-1-yl)-1-piperazine;
(-)-4-(6-Methoxy-indan-1-yl)-1-[(1-methyl-pyrrol-2-yl)carbonyl]piperazine;
(-)-1-[(2-Furanyl)carbonyl]-4-(6-methoxy-indan-1-yl)piperazine;
(-)-4-(6-Methoxy-indan-1-yl)-1-[(3-methyl-furan-2-yl)carbonyl]piperazine;
(-)-1-(2-Hydroxybenzoyl)4-(6-methoxy-indan-1-yl)piperazine;
(-)1-[(3-Bromothien-2-yl)carbonyl]-4-(6-Methoxy-indan-1-yl)piperazine;
(-)-4-(6-Methoxy-indan-1-yl)-1-[(1-methyl-thien-2-yl)carbonyl]piperazine;
(-)-4-(6-Methoxy-indan-1-yl)-1-[(3-thienyl)carbonyl]piperazine; and
1-(Cyclobutylcarbonyl)-4-(6-methoxy-indan-1-yl)piperazine.

5. The compound of claim 2 wherein R¹ is NR²R³.

6. The compound of claim 5 selected from the group consisting of:
(-)-N-Ethyl-4-(6-methoxy-indan-1-yl)piperazine-1-carboxamide;
N-Methyl-4-(6-methoxy-indan-1-yl)piperazine-1-carboxamide;
N-(n-Propyl)-4-(6-methoxy-indan-1-yl)piperazine-1-carboxamide;
N-Ethyl-4-(6-methoxy-indan-1-yl)piperazine-1-carboxamide;
N-(1-Methylethyl)-4-(6-methoxy-indan-1-yl)piperazine-1-carboxamide;
N-Cydopropyl-4-(6-methoxy-indan-1-yl)piperazine-1-carboxamide; and
N-Cyclopropyl-4-(6-methoxy-indan-1-yl)piperazine-1-carboxamide.

7. The compound of claim 2 wherein R¹ is C₂₋₄ alkenyl.

8. The compound of claim 7 selected from the group consisting of:
1-(Ethenylcarbonyl)-4-(6-methoxy-indan-1-yl)piperazine; and
1-[(1-Methyl-ethenyl)carbonyl]-4-(6-methoxy-indan-1-yl)piperazine.

9. The compound of claim 2 wherein R¹ is C₁₋₆ alkyl.

10. The compound of claim 9 selected from the group consisting of:
(-)4-(6-Methoxy-indan-1-yl)-1-(2-methylthioacetyl)piperazine;
4-(6-Methoxy-indan-1-yl)-1-(2-methyl-propanoyl)piperazine;
1-Butanoyl-4-(6-methoxy-indan-1-yl)piperazine;
1-(2,2-dimethyl-propanoyl)-4-(6-methoxy-indan-1-yl)piperazine; and
(-)4-(6-Methoxy-indan-1-yl)-1-(2-methyl-propanoyl)piperazine.

11. The compound of claim 1, (-)-N-Ethyl-4-(6-methoxy-indan-1-yl)piperazine-1-carboxamide.

12. The compound of claim 1, (-)-1-(Cyclopropylcarbonyl)-4-(6-methoxy-indan-1-yl)piperazine.

13. The compound of claim 1, (-)-4-(6-Methoxy-indan-1-yl)-1-[(2-thienyl)carbonyl]piperazine.

14. The compound of claim 1, 1-(Cyclopropylcarbonyl)-4-(6-methoxy-indan-1-yl)piperazine.

15. The compound of claim 1, 4-(6-Methoxy-indan-1-yl)-1-(2-methyl-propanoyl)piperazine.

16. The compound of claim 1, 1-(Cyclopropylcarbonyl)-4-(6-ethoxy-indan-1-yl)piperazine.

17. The compound of claim 1 wherein m = 2 selected from the group consisting of:
1-Acetyl-4-(7-methoxy-tetralin-1-yl)piperazine;
N-Methyl-4-(7-methoxy-tetralin-1-yl)piperazine-1-carboxamide;
1-(Cyclopropylcarbonyl)4-(7-methoxy-tetralin-1-yl)piperazine;
1-(1-Oxobutyl)-4-(7-methoxy-tetralin-1-yl)piperazine; and
N-Ethyl-4-(7-methoxy-tetralin-1-yl)piperazine-1-carboxamide.

18. The compound of claim 1 selected from the group consisting of:
1-(Cyclopropylcarbonyl)-4-(6-methoxy-indan-1-yl)homopiperazine;
N-Ethyl-4-(6-methoxy-indan-1-yl)homopiperazine-1-carboxamide; and
4-(6-Methoxy-indan-1-yl)-1-(2-methyl-propanoyl)homopiperazine.

19. A pharmaceutical composition for treating a sleep or circadian rhythm disorder in a patient in need of such treatment comprising an effective amount of a compound of claim 1 and a suitable amount of a pharmaceutically acceptable carrier.

20. Use of a compound according to claim 1 in the manufacture of a medicament for treating a sleep disorder in a patient in need of such treatment.

21. A pharmaceutical composition for treating depression in a patient in need of such treatment comprising an effective amount of a compound of claim 1 and a suitable amount of a pharmaceutically acceptable carrier.

22. Use of a compound according to claim 1 in the manufacture of a medicament for treating a depression in a patient in need of such treatment.

23. A process of making an indanyl piperazine or homopiperazines of formula I-i wherein R^{a} is H, C₁₋₄ alkyl, C₁₋₉ alkoxy, or halogen; and n is 1 or 2, which comprises the steps of :
(1) condensing an indanone of formula VI 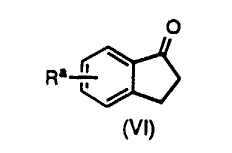 with a piperazine, homopiperazine, N-formyl piperazine, or N-formyl homopiperazine in the presence of titanium (IV) isopropoxide to form a titaniumlindanone/piperazine complex;
(2) reducing the complex produced in step (1) with sodium borohydride, sodium cyanoborohydride in a suitable solvent;
(3) quenching the reaction using aqueous base; and
(4) recovering the formula I-i product.

24. The process of claim 23 wherein the product of step (1) is hydrogenated in the presence of a suitable catalyst before step (3).

25. The process of claim 23 wherein n is 1 and the product is a piperazine.
